# EUROPEAN PATENT SPECIFICATION

(11) **EP 2 852 394 B1**
(45) Date of publication and mention of the grant of the patent: **20.02.2019**
(21) Application number: 13723573.5
(22) Date of filing: 27.03.2013
(51) Int. Cl.: A61K 35/74, A61K 39/02

(54) **ORAL VACCINE CONTAINING THE BACILLUS SUBTILIS SPORES AND ITS APPLICATION TO IMMUNISE AGAINST HELICOBACTER PYLORI**
ORALER IMPFSTOFF MIT BACILLUS-SUBTILIS-SPOREN UND DESSEN VERWENDUNG ZUR IMMUNISIERUNG GEGEN HELICOBACTER PYLORI
VACCIN ORAL CONTENANT DES SPORES DE BACILLUS SUBTILIS ET SON APPLICATION POUR IMMUNISER CONTRE L'HELICOBACTER PYLORI

(30) Priority: 29.03.2012 PL 39865812
(43) Date of publication of application: 01.04.2015
(73) Proprietor: Gdanski Uniwersytet Medyczny, 80-210 Gdansk (PL)
(72) Inventor: HINC, Krzysztof, 80-276 Gdansk (PL); OBUCHOWSKI, Michal, 80-299 Gdansk (PL); RICCA Ezio, 80129 Naples (IT)
(86) International application number: PCT/PL2013/000040
(87) International publication number: WO 2013/147628

(56) References cited:
- Hink K: "Expression and display of UreA of Helicobacter acinonychis on the surface of Bacillus subtilis spores.", Microb. Cell Fact. , vol. 9:2 18 January 2010 (2010-01-18), pages 1-11, XP002713106, Retrieved from the Internet: URL:http://www.microbialcellfactories.com/ content/9/1/2 [retrieved on 2013-09-12]
- GOMEZ-DUARTE O G ET AL: "Protection of mice against gastric colonization by Helicobacter pylori by single oral dose immunization with attenuated Salmonella typhimurium producing urease subunits A and B", VACCINE, ELSEVIER LTD, GB, vol. 16, no. 5, 1 March 1998 (1998-03-01), pages 460-471, XP004106959, ISSN: 0264-410X, DOI: 10.1016/S0264-410X(97)00247-8
- JUNEHYUNG KIM ET AL: "Display of proteins on Bacillus subtilis endospores", CMLS CELLULAR AND MOLECULAR LIFE SCIENCES, BIRKHÄUSER-VERLAG, BA, vol. 66, no. 19, 25 June 2009 (2009-06-25) , pages 3127-3136, XP019755977, ISSN: 1420-9071, DOI: 10.1007/S00018-009-0067-6
- KASAHARA T. ET AL.: "Interleukin 2-mediated immune interferon (IFN-gamma) production by human T cells and T cell subsets", J. IMMUNOL., vol. 130, 1983, pages 1784-1789,

## Description

### Field of the invention

Described herein are oral vaccines containing *Bacillus subtilis* spores to serve as the antigen carrier, and UreA or UreB proteins of the *Helicobacter pylori* or *Helicobacter acinonychis* urease serving as the antigens. The antigens are fused with coat proteins CotB, CotC, CotG, or CotZ to express them on the surface of the B. subtilis spore. The vaccines are useful to prevent/ treat *Helicobacter pylori* infections,

### Background of the Invention

*Helicobacter pylori* (*H.pylori*) infections are common world wide, although the size of the affected population differs from country to country. The development of *H. pylori* infection in the stomach leads to gastric mucosa inflammation, mainly in the prepyloric part, and may then spread onto the stomach. At a further stage, approximately 80-90% of the infected persons develop chronic gastric mucosa inflammation. In long-lasting infections chronic inflammation may lead to atrophy of the mucosa (the so-called atrophic inflammation). Aggravated inflammation may cause mucosa defects called erosive gastritis or even lead to haemorrhages (haemorrhagic inflammation). Moreover, *H. pylori* infections are seen as the main cause of the chronic duodenal and gastric ulcer disease.

In *H. pylori-infected* persons, there is a correlation observed between the presence of the bacteria and the occurrence of early cancer of the stomach http://pl.wikipedia.org/wiki/Helicobacter pylori - cite note-48 The World Health Organisation has found *H. pylori* a factor of proven carcinogenic effects.

Sometimes *H. pylori* infections may lead to uncontrolled proliferation of the lymphatic system tissue in the stomach - the so-called MALT lymphoma (Mucosa Associated Lymphoid Tissue). The complication of the type is a particularly acute problem in the so-called developing countries, especially China and India, where the infection affects up to 80-90% of the population.

Although *H. pylori* infections are common worldwide, the difference in the size of the infected population is substantial between highly developed and developing countries. On the country-wide level, the distribution of the infection cases within the infected population differs by age. In general, the occurrence of *H. pylori* infection is strongly correlated with socioeconomic conditions. While more than 80% of middle-aged individuals in developing countries suffer from the infection, only 20 to 50% of the same age group population are infected in the highly developed countries.

The publication by Duc and associates of 2003 describes methods of using the spores for the presentation of a tetanus toxin fragment (*Clostridium tetani*) administered intragastrically to laboratory animals (mice). A cycle of immunisation with recombined spores resulted in the development of resistance to tetanus toxin, thanks to which 50% of the animals which had received 20 times the LD₅₀ dose survived. The obtained results unquestionably prove that the spores may be effectively used as macroparticles for antigen presentation in oral immunisation.

Patent description WO 2008/040155 (patent EP 2 082 750 B) disclosed a recombined protein used for immunoprofilaxy of *Helicobater pylori* infections in humans. Specifically claimed is the recombined protein formed by combination of A2 and B subunits of heat-labile *Escherichia coli* entherotoxic enetotoxin with B subunits of *Helicobacter pylori* urease. The pharmaceutical preparation takes the form of slow release microspheres.

Patent description WO 94/09823 (PL 174448 B) disclosed a *Helicobacter pylori* infection vaccine which contained *H.pylori* or *H. felis* urease, or a polypeptide containing its subunit. It also contained a mucosal adjuvant (cholera toxin).

Similarly, disclosed in publication WO 95/22987 (PL 179149 B) is a vaccine against *Helicobacter pylori* infection, which contains *H.pylori* or *H. felis* urease, or a polypeptide containing its subunit. Moreover, it contains a mucosal adjuvant in the form of heat-labile *Escherichia coli* toxin or a polypeptide containing its subunits or fragments.

One of the key factors which prevent proposing an effective vaccine is the fact that the place where the bacteria colonise in the human body is the stomach. Its acid environment kills the antibodies, which makes the traditional immunisation methods evoking humoral response fail.

Creating an effective vaccine against *Helicobacter pylori* infections is one of the challenges of the contemporary medicine. *H. pylori* is responsible for ulcer disease, it is also believed to lead to stomach cancer.

It has been suggested to use Urease proteins as immunogens in an attempt to create vaccines against H. pylori (Gomez-Duarte (1998); Vaccine 16(5):460-471).

However, no successful vaccine is yet available.

A treatment method commonly used today is an antibiotic therapy. It entails, however, many disadvantageous effects. Antibiotic therapy is burdensome and carries many highly undesirable side effects. One of them is the development of bacteria strains resistant to the specific antibiotic. The fact is extremely important since it makes it necessary to use ever new antibiotics, while many specialists claim the arsenal of adequate antibiotics at disposal in medicine is exhausting. Moreover, the therapy of the type used today is time consuming, which is highly undesirable in the case of antibiotics and causes additional complications. A serious disadvantage of the antibiotic therapy consists in its short-lived effects and the resulting relapses of *H. pylori* infections.

As at today, there is no vaccine against *H. pylori* infections available, and the therapy at hand comes down to administering antibiotics.

The National Institute of Health, USA, registered 104 clinical trials on *Helicobacter.* Absolute majority of the trials concern treatment with two, three, or any larger number of antibiotics. None of the registered clinical trials seems to have been conducted to the purpose of registering a vaccine against *Helicobacter pylori.* Some of the trials and active tests were discontinued for various reasons, e.g. 69 trials/ tests were abandoned in phases I to IV, with 8 discontinued in phase I, 17 in phase II, 18 in phase III, and 26 in phase IV.

### Basis of the invention and general disclosure

The following disclosure provides the general basis for the invention and contemplates the use of UreA and/or UreB of Helicobacter in a vaccine against H. pylori.

The invention claimed, however, concerns a vaccine with UreB3, which is illustrated (but not limited) in the examples with spores BHK108 combined with BHK121.

As for the basis for the invention, it has been found that *Bacillus subtilis* spores may be used as peculiar antigen carriers for antigens, where the antigens are presented on the spore surface as fusion proteins with natural coat components - the external building structure of the B. subtilis spores (Hinc (2010); Microb. Cell. Fact. 9:2).

We now propose to create vaccines against H. pylori using such spores expressing. *H. acinonychis* UreA and UreB proteins as antigens (bacterial urease subunits) *Helicobacter acinonychis* proteins have been proposed for application considering that in genetic terms *H. pylori* differs from *H. acinonychis* by a mere 8%, which makes it a convenient model for tests on animal models. The obtained results should be identical when the method is used on the human *H. pylori.*

The basis of the invention consists in using the recombined spores of the globally found non-pathogenic *Bacillus subtilis* soil bacterium as carriers of the oral vaccine against infections caused by *Helicobacter pylori.*

To that end, selected UreA and UreB proteins (in the role of antigens) - subunits of the bacterial urease of the *Helicobacter* type - will be presented on the spore surface as fusion proteins with Cot proteins - natural components of the coat, i.e. the external structure building the spore. The immunisation trials were based on the use of both the whole UreA and UreB proteins, and those fragments thereof which were most likely to contain the antigen epitopes. The fusion proteins were prepared with the use of integration vectors. The vectors contain CotB, CotC, CotG and CotZ coding genes copied under the PCR method together with the promoter regions. Moreover, restriction sites were introduced during the PCR reaction, which facilitated both the process of cloning to the vectors, and the formation of the fusion proteins. The obtained fusion genes were subject to transformation to *B. subtilis* cells, where upon the occurrence of double crossing-over they were incorporated into *locus amyE, thrC* or *lacA.* The obtained fusion genes are controlled by the physiological *B. subtilis* promoter activated exclusively at late stages of the sporulation process. The formed fusion proteins were used in the formation of the coat, thanks to which they are present on the spore surface.
Using the spores as vaccine carriers constitutes a new approach to immunisation of humans and animals and may found significant application particularly in immunisation aimed at providing protection against infections of the gastrointestinal tract. In this case oral administration of the vaccine enables achieving both mucosal, and systemic immunity and extension of the half-life of the administered antigen. An unquestionable advantage of the *B. subtilis* spores used as the vaccine carrier is that they demonstrate adjuvant effects, which manifests itself in enhancing the cellular-type immune response (Th1). The fact can be of significance for vaccine effectiveness.

Moreover, using the *B. subtilis* spores as the antigen carriers facilitates the production of endospores - *B. subtilis* is a bacterium of low trophic requirements, which translates to a relatively low cost of producing the spores in industrial quantities. Moreover, spore production is a natural process and does not require human intervention in bacterial physiology.

Omnipresence of spores in the natural environment, human food included, testifies to their low immunogenicity, which will prevent the development of allergic reactions during administration of the antigen-presenting spores.

High resistance of the spores to various environmental conditions enables their easy storage without the need to freeze them or keep the cold chain.

The possibility of mixing spores presenting different antigens enables producing many quantitative and qualitative vaccine combinations. Moreover, it enables instant preparation of the desired antigen epitope combination by mixing recombined spores in the proper proportions in the standard laboratory conditions.

Administration of spore-containing vaccines may bring about additional positive health effects, since the *B. subtilis* spores are used as probiotics, and their modification so that they can present antigens will not reduce their beneficial effects on the gastrointestinal tract of the immunised animals or humans.

Preparation of the *B. subtilis* spores containing fragments of UreA and/or UreB proteins in the form of fusion proteins with e.g. CotB, CotC, CotG, or CotZ. The thus prepared material is administered orally to the patient and in this way delivered to its destination, i.e. the stomach. The spore and its inherent resistance to digestion protects the antigens which will then be able to stimulate the immune response against *H. pylori,* thus building immunity of the organism to that particular pathogen.

The creation of a specific drug to treat *H. pylori* infections is hindered because the pathogen has an adapting mechanism, enabling e.g. expression of antigens structurally similar to the carrier - bacterial LPS and antigens of the Lewis group found on gastric mucosal cells. The antigen mimicry may lead to effective extinguishment of the specific immune response.

An additional obstacle to the development of an effective vaccine comes from the environment in which *H. pylori* colonises the body, i.e. the stomach. This is where the food is digested, and as such its environment may be considered 'caustic'. Moreover, the same digestion process makes the traditional immunisation methods futile in terms of their therapeutic and protective effects.

The method proposed herein enables the vaccine to 'survive' in the hostile environment of the stomach, and eliminate the need to resort to antibiotic therapy.

The fundamental benefit of the vaccine will consist in building immunity to *H. pylori* infection and re-infection among the convalescents. This will enable to reduce the number of new infections substantially, and thus reduce the costs of treating the infected patients. An additional tangible benefit of using the vaccine will consist in the possibility to abandon antibiotic therapy to the extent it has been resorted to so far. In effect, the process of developing drug resistance by *H. pylori* will be slowed down. This is even more important if one bears in mind that many physicians and scientists note that the possibilities of antibiotic therapy are getting gradually exhausted because of the development of multidrug resistant strains. This means that in the relatively near future we may come to the point when it will not be possible to treat the infection effectively since the bacterium will be resistant to all available antibiotics. In approximately 50% cases *H. pylori* infections lead to the development of the stomach cancer. The global population of patients suffering from this type of cancer is estimated at over 970 000 cases a year, which represents ca. 10% of all cancer incidents. An effective vaccine against *H. pylori* will enable attaining a substantial reduction in the number of new stomach cancer incidents. Moreover, the invention has the potential of being used for therapeutic purposes in the treatment of already infected patients. The possibility of therapeutic use of the vaccine carries many advantages, such as the substantial limitation of the antibiotic therapy and the side effects thereof, as well as the related financial benefits.

The form in which the vaccine/ drug is taken will offer yet another benefit. The goal of the invention is to obtain an orally administered preparation and avoid such medical procedures as injections. Such solution is advantageous to both the patient, and the 'health care system', particularly in the developing countries where medical disposables are rarely used (needles in particular), which fosters other infections. The possibility to abandon using the disposables (needles, syringes, antiseptics) will allow reducing the costs of immunising large groups of patients. Moreover, oral administration of the vaccine will mitigate the irrational fear of injections, which causes that a part of the population postpones the vaccination to a later unspecified date. Thanks to the use of bacterial spores as antigen carriers the ready vaccine preparation will not require storing in any specific conditions, such as low temperature, which will facilitate its transport and enable long storage (a few to an odd dozen years). The patient will be able to buy the vaccine in the form of e.g. powder or suspension and take it at any time of his/her choice (e.g. before, or after the meal). Yet another benefit of using the vaccine in development will come down to the fact the *B. subtilis* bacteria for years used as a probiotic helping to regulate the functioning of the gastrointestinal tract will be introduced into the tract of the treated patients.

Thus, disclosed herein are oral vaccines containing the *Bacillus subtilis* spores which serve as the antigen carrier, and UreA or UreB proteins of the *Helicobacter pylori* or *Helicobacter acinonychis* urease serving as the antigen fused to the spore coat CotB, CotC, CotG, CotZ proteins of the spore coat.

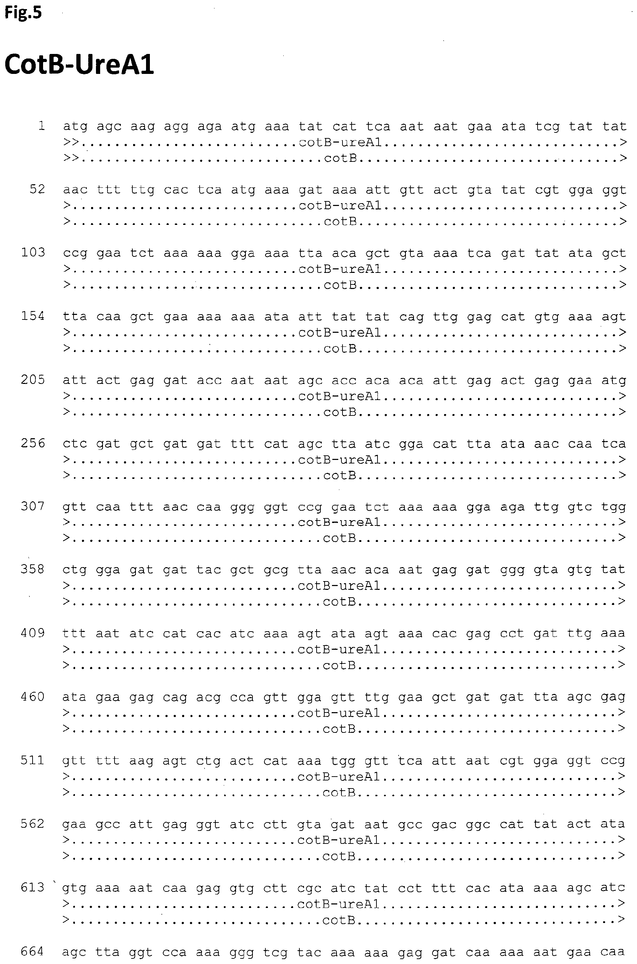

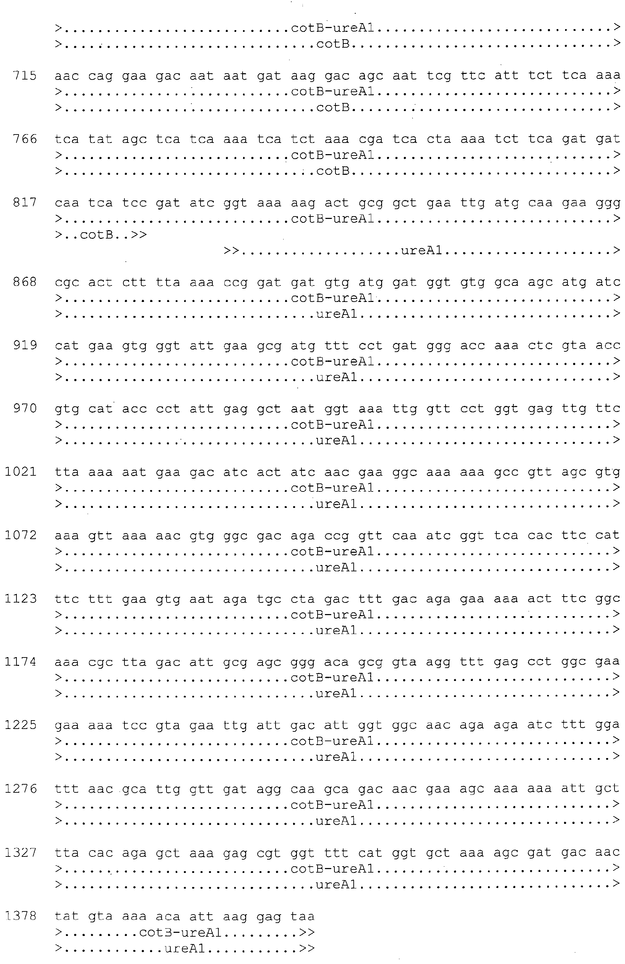

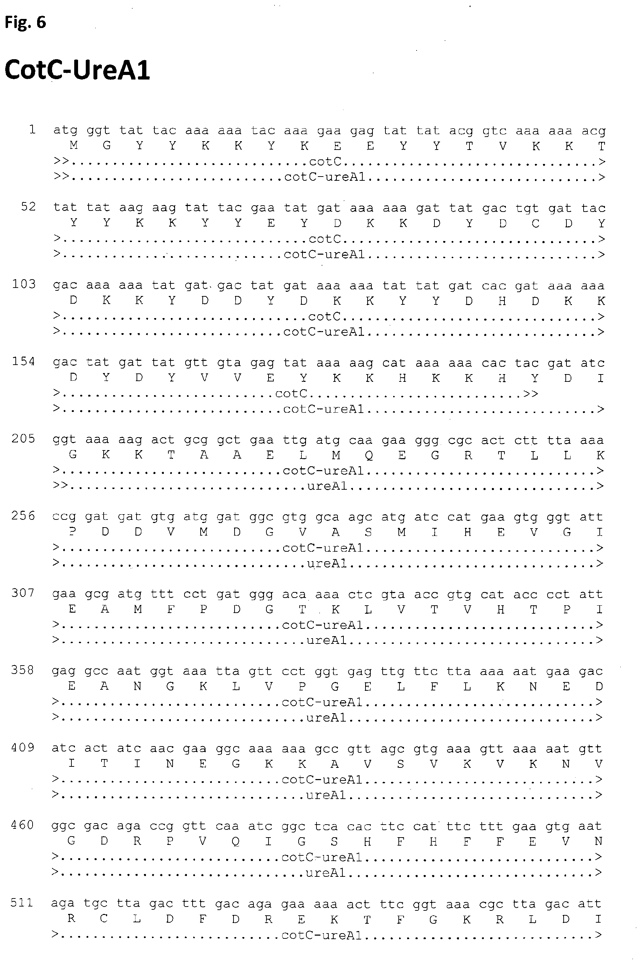

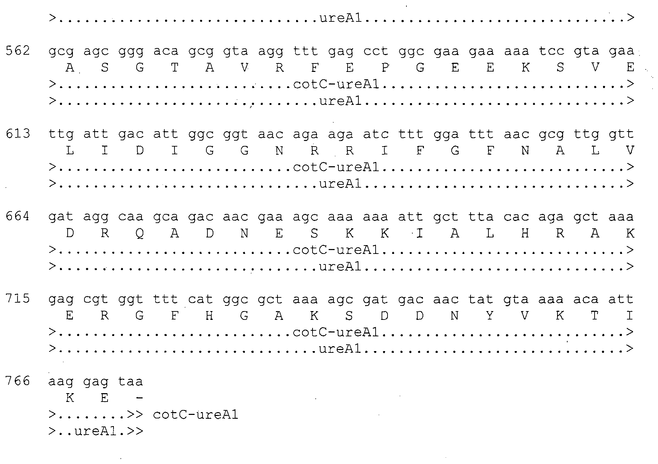

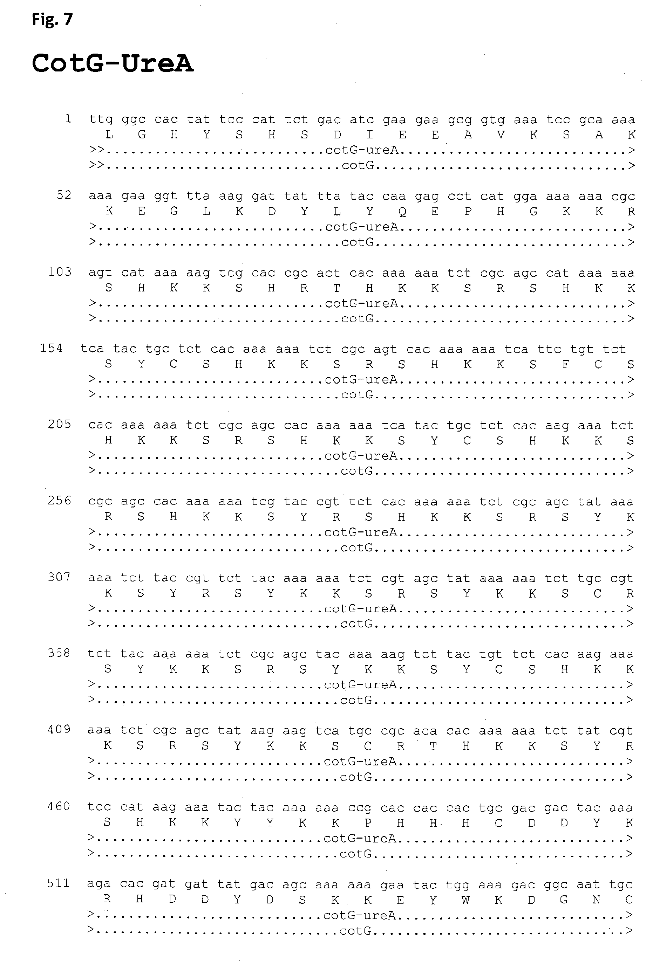

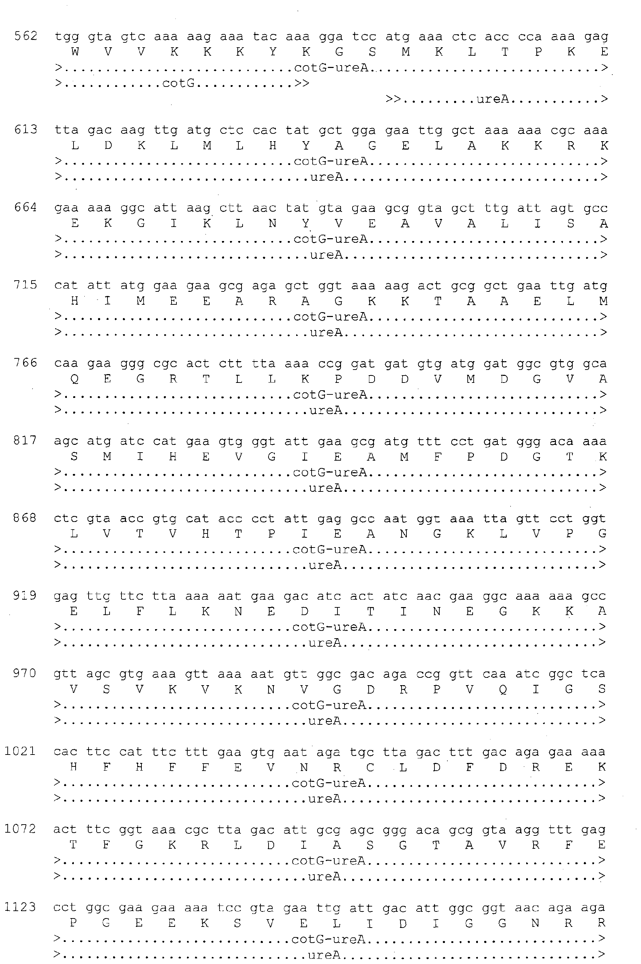

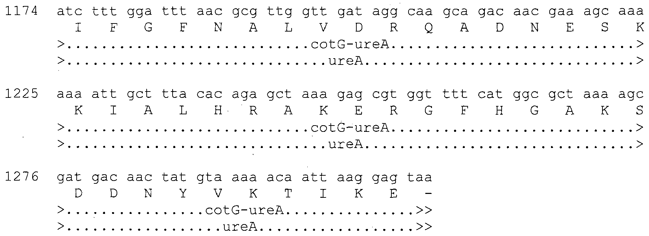

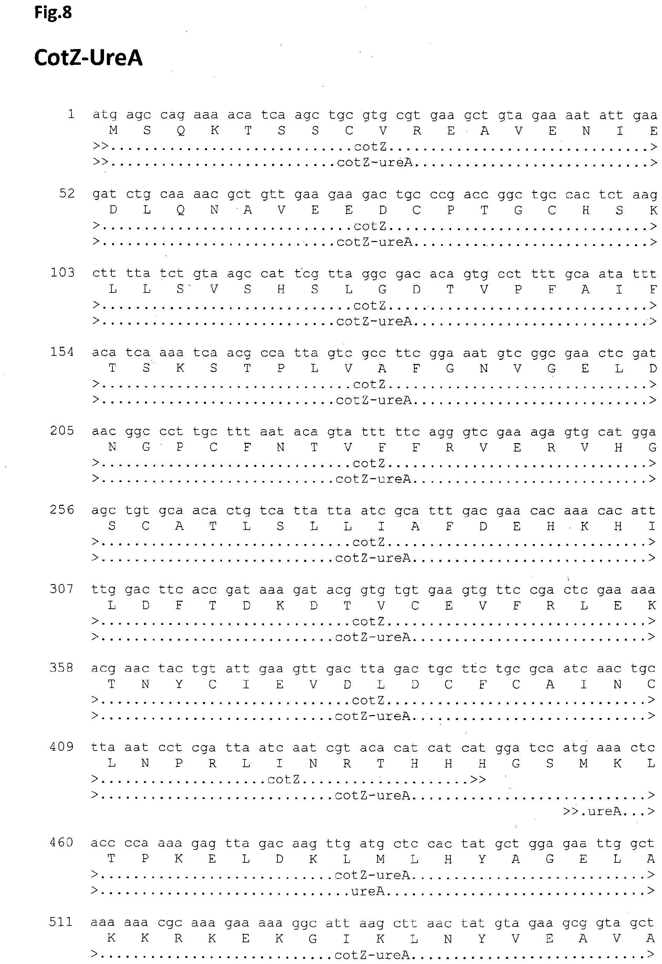

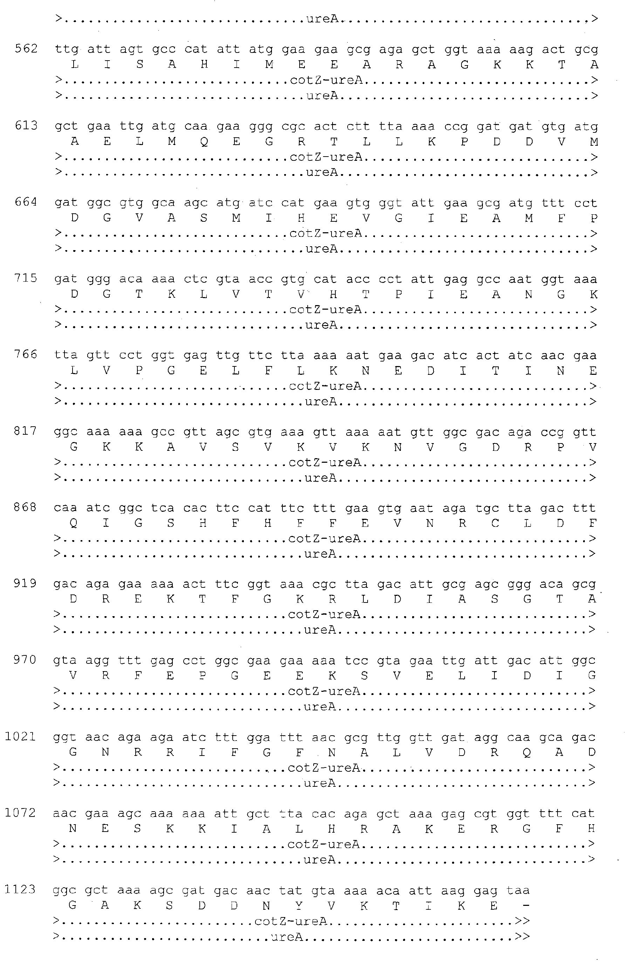

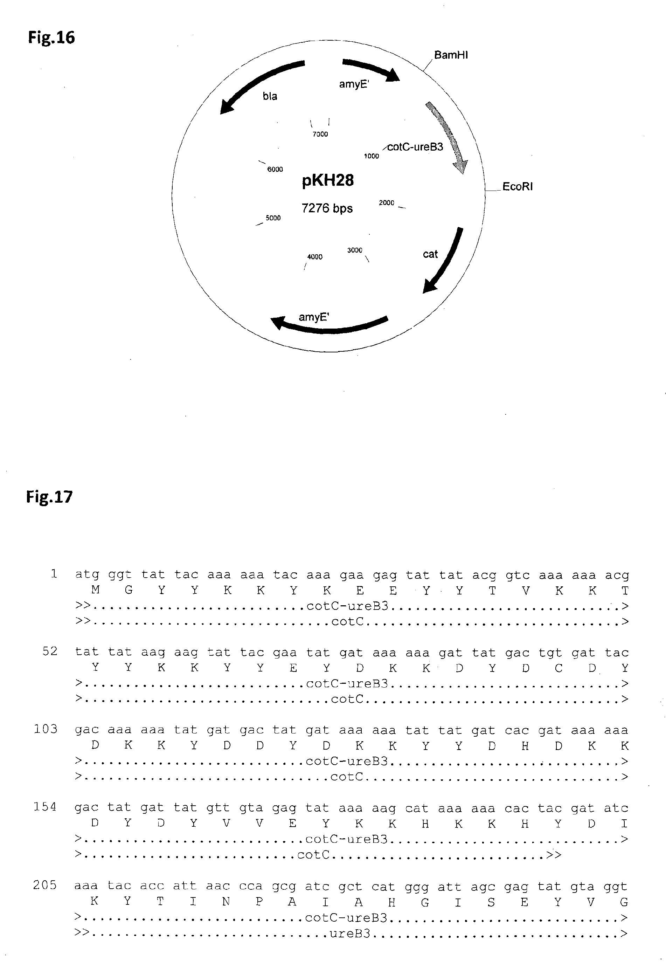

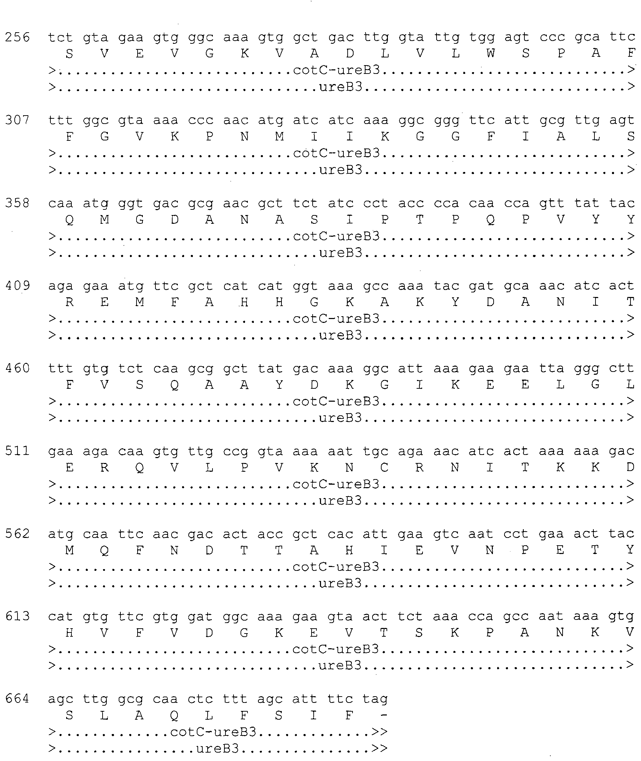

Sequences of exemplary fusions of Cot and Ure proteins are:
CotB fused to UreA as shown in Fig. 5,
CotC fused to UreA shown in Fig. 6
CotG fused to UreA as shown in Fig. 7,
CotZ fused to UreA as shown in Fig. 8,
CotB fused to UreB as shown in Fig. 17.

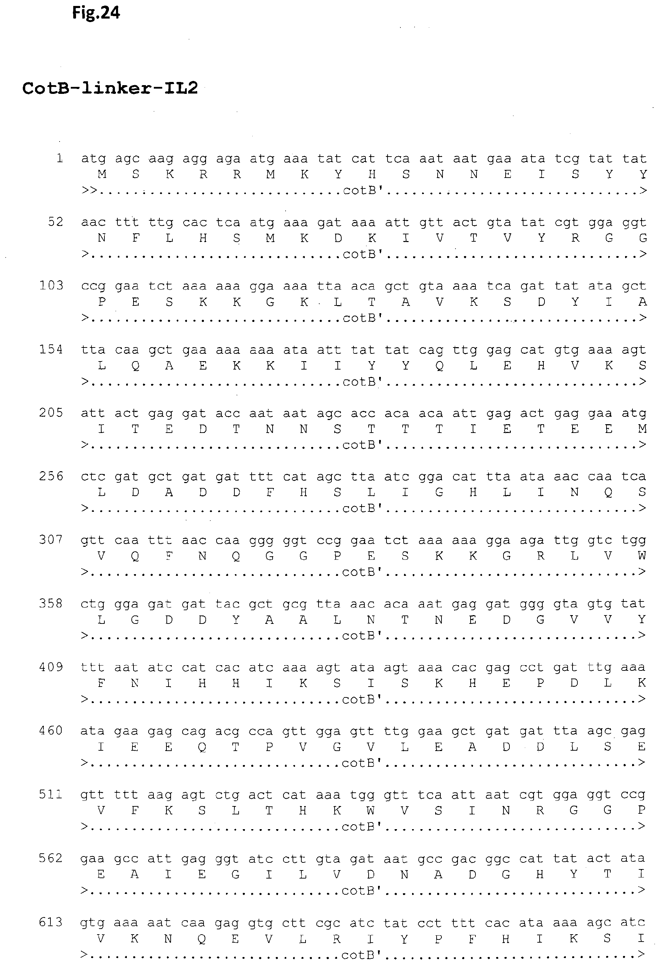

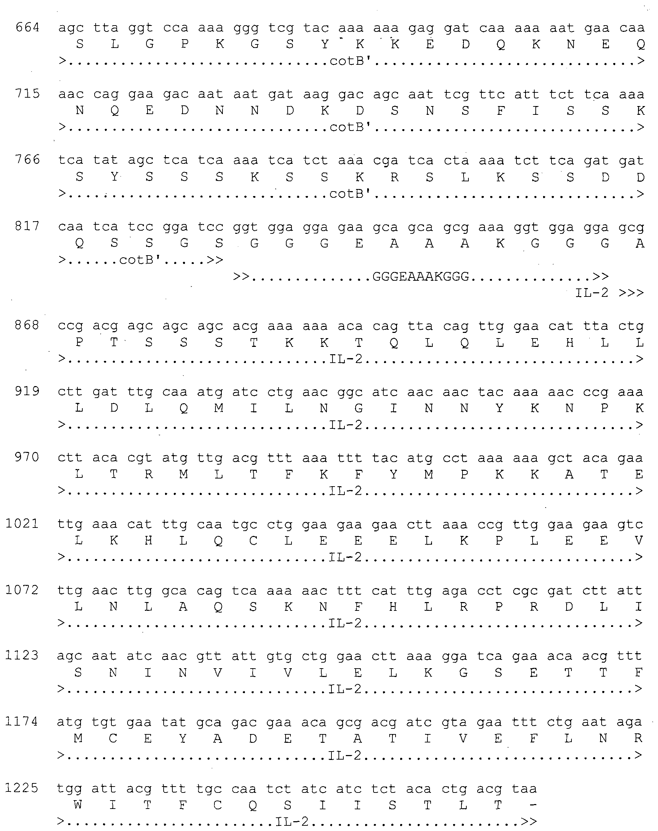

Further provided in Figure 24 is the sequence of IL-2, which can be used as adjuvant in the vaccine.

The disclosed *Bacillus subtilis* spores can be used to produce the oral vaccine, which in a therapeutically effective dose, can be administered to immunise patients suffering from the duodenal and gastric ulcer disease against *Helicobacter pylori.*

The advantages of implementing the invention are as follows:
- the possibility to immunise large populations and thus to eliminate or substantially reduce the occurrence of *H. pylori* infections and the development of ulcer disease or stomach cancer;
- easy administration of the vaccine produced as an orally administered suspension;
- the possibility to store the vaccine/ drug in normal conditions without the need to keep any specific environment.
- limited application of antibiotic therapy,
- reduction of the therapy costs.

### Definitions

The terms used above, as well as in the patent description and claims are defined as follows:
**Oral vaccine** - enterally-administered preparation evoking a specific immune response in the recipient organism.
The Bacillus subtilis strain - strain No. 168 (wild laboratory strain)
Helicobacter pylori - strain No. NTC 11637
Helicobacter acinonychis - strain No. ATCC 51101

### Description of the figures:

**Fig.1** - shows the fusion proteins UreA-CotB recognised by antibodies directed against both CotB, and UreA proteins.
**Fig.2** - shows the fusion proteins UreA-CotC recognised by antibodies directed against both CotC, and UreA proteins.
**Fig.3** - shows the fusion proteins UreA-CotG recognised by antibodies directed against both CotG, and UreA proteins.
**Fig.4** - presents quantification of the fusion proteins UreA-CotC presented on the surface of the recombined spores formed in the strains of different genetic backgrounds. 1 - purified UreA protein, 2 - extract of the proteins of the wild-type spore coat, 3 - extract of the *cotB::ureA1* strain spores, 4 - extract of the *cot::ureA1* strain spores, 5 - extract of the *cotC::spc cotC::ureA1* strain spores, 6 - extract of the *cotC::spc cotU::erm cotC::ureA1* strain spores, 7 - extract of the *cotG::urea* strain spores, 8 - extract of the *cotZ::urea* strain spores.
**Fig.5** - shows the nucleotide and amino acid sequences of the recombined CotG gene and UreA protein or its fragments.
**Fig.6** - shows the nucleotide and amino acid sequences of the recombined CotC gene and UreA protein or its fragments.
**Fig.7** - shows the nucleotide and amino acid sequences of the recombined CotB gene and UreA protein or its fragments.
**Fig.8** - shows the nucleotide and amino acid sequences of the recombined CotZ gene and UreA protein or its fragments.
**Fig.9** - presents the maps of the following integration vectors: pKH20 (A), pKH03 (B), pKH14 (C), pKH102 (D). Expression of the recombined genes is ensured by the following respective physiological gene promoters: *cotG*, *cotC*, *cotB* and *cotZ.*

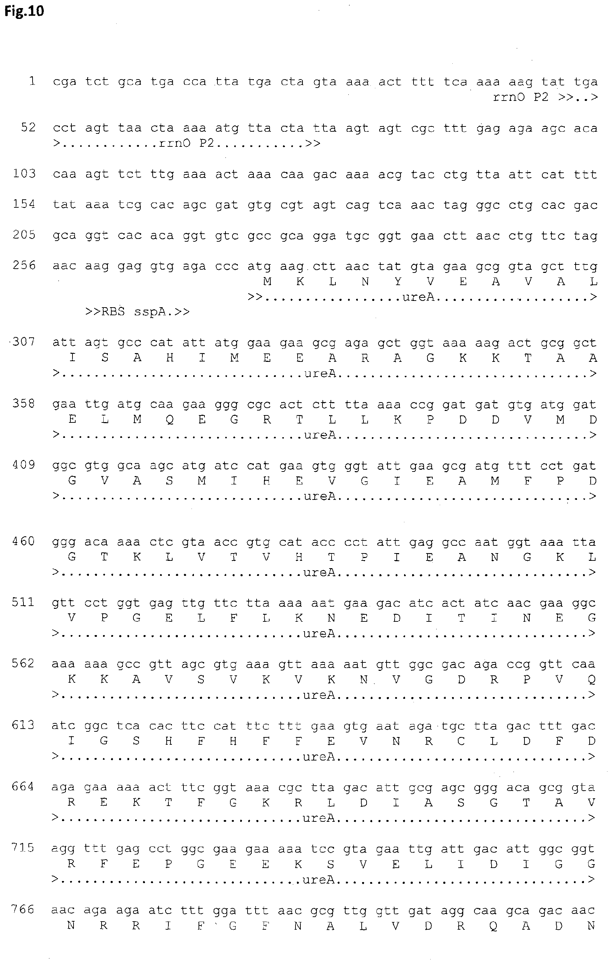

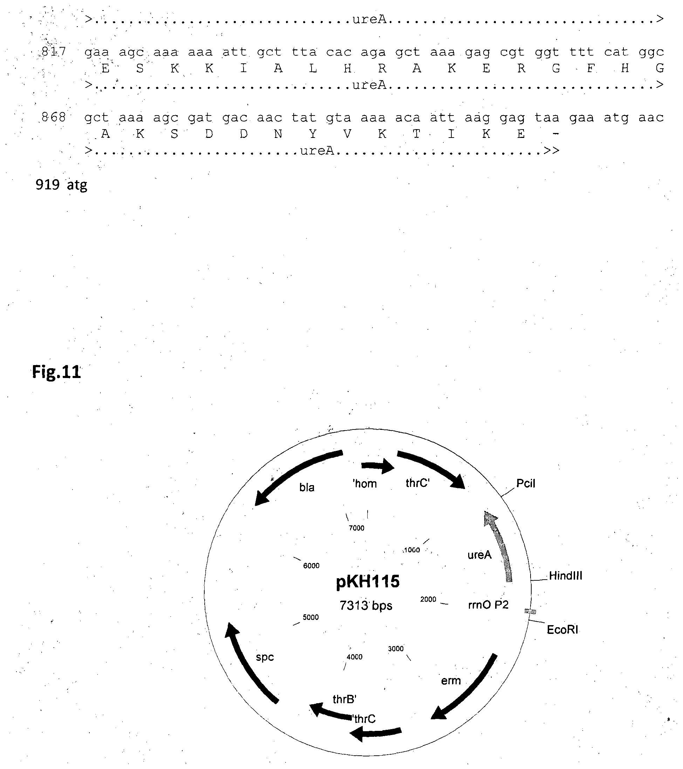

**Fig.10** - shows the nucleotide sequence of the rrnOP2 promoter, and the nucleotide and amino acid sequences of the UreA gene and protein.
**Fig.11** - shows integration vector pKH115 containing the *ureA* gene controlled by the rrnO P2 ribosomal promoter.

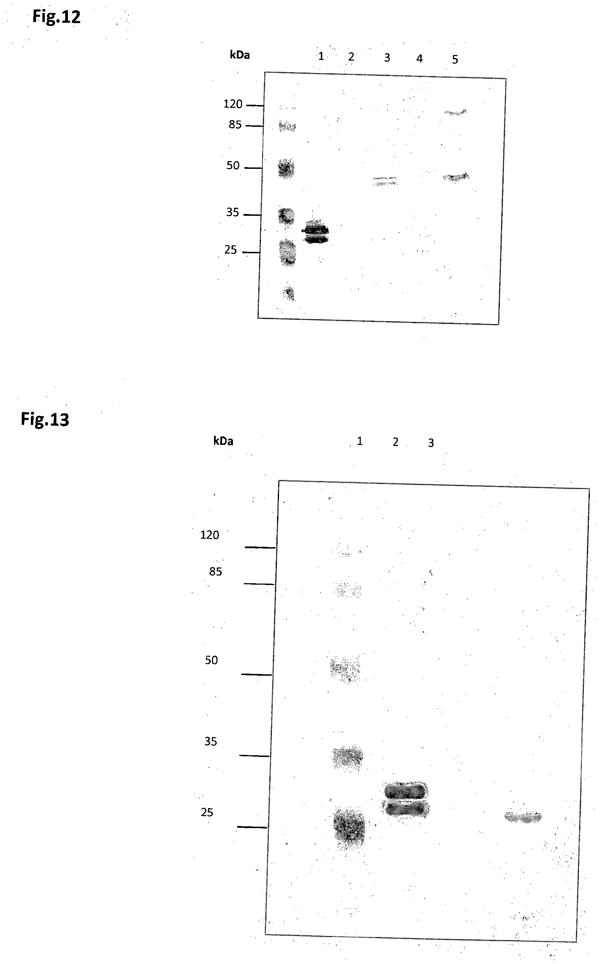

**Fig.12** - presents Western Blotting with the use of protein extracts obtained from the spore coat. The samples applied: 1- purified UreA, 2- 168 (wild-type strain), 3- BKH102 (*amyE:: cotZ-ureA*), 4- BKH115 (*thrC:: rrnOP2* - *ureA*), 5- BKH116 (*amyE:: cotZ-ureA, thrC:: rrnOP2 - ureA).*
**Fig.13** - presents Western Blotting with the use of protein extracts obtained from vegetative cells. The samples applied: 1- purified UreA, 2- 168 (wild-type strain), 3- BKH116 (*amyE:: cotZ-ureA, thrC:: rrnOP2* - *ureA*).
**Fig.14** - shows the immunofluorescences using spores of the BKH102 strain purified through rinsing with detergents. The experiment used mouse anti-UreA primary antibodies and anti-mouse IgG-Cy3 secondary antibodies which enabled their visualisation in the fluorescence microscope. The left photograph shows the image seen in the visible light (phase contrast), while the right presents the fluorescent image of the same field of vision.
**Fig.15** - shows the results of the ELISPOT assay. Each spot is the 'footprint' of a single cell that has secreted IFN-γ in response to the present antigen. Splenocytes were isolated from non-immunised mice (blue), those immunised with wild-type spores - 168 (red), and yet others immunised with BH102 presenting the antigen (green).
**Fig.16** - presents map of the pKH28 integration vector. Expression of the recombined gene is ensured by the physiological promoter of the CotC gene.
**Fig.17** - shows the nucleotide and amino acid sequences of the recombined gene and the CotC-UreB3 protein.
**Fig.18** - shows Western Blotting with the use of protein extracts obtained from the spore coat. The samples applied:1 - 168 (wild-type strain), 2- BKH28 (*amyE:: cotC-ureB3), 3-*RH101 (*cotC:: spc*) 4- BKH48 (*cotC:: spc*, *amyE:: cotC-ureB3*), 4- BKH48 (*cotC:: spc*, *amyE:: cotC-ureB3*), 5- RH209 (*cotC..spc cotU::erm*), 6- BKH49 (*cotC:: spc*, *cotU::erm*, *amyE:: cotC-ureB3*).
**Fig.19** - shows the immunoflorescences using spores of the BKH28 strain purified through rinsing with detergents. The experiment used mouse anti-UreB primary antibodies and anti-mouse IgG-Cy3 secondary antibodies which enabled their visualization in the fluorescence microscope. The left photograph shows the image seen in the visible light (phase contrast), while the right depicts the fluorescent image of the same field of vision.
**Fig.20** - shows the pKH101 integration vector containing the *ureB* gene controlled by the rrnO P2 ribosomal promoter.
**Fig.21** - presents the nucleotide sequence of the rrnOP2 promoter and the nucleotide and amino acid sequences of the UreB gene and protein.
**Fig.22** - shows Western Blotting with the use of protein extracts obtained from the endospore coat (1 and 2) and vegetative cells (3 and 4). The samples applied: (1 and 3) - 168 (wild-type strain), (2 and 4)- BKH108 (*amyE:: cotC-ureB3, thrC:: rrnOP2* - *ureB*).
**Fig.23** - presents the map of the pKH124 integration vector. Expression of the recombined gene is ensured by the physiological promoter of the *CotB* gene.
**Fig.24** - shows the nucleotide and amino acid sequences of the recombined CotB-linker-IL-2 gene and protein.
**Fig.25** - presents the CotB-linker-IL-2 fusion protein recognised by the antibodies directed against both the CotB protein and the IL-2 protein.
**Fig.26** - shows the immunoflorescences using spores of the BKH121 strain purified through rinsing with detergents. The experiment used mouse anti-IL-2 primary antibodies and anti-mouse IgG-Cy3 secondary antibodies which enabled their visualization in the fluorescence microscope. The left photograph shows the image seen in the visible light (phase contrast), while the right depicts the fluorescent image of the same field of vision.
**Fig.27** - shows the results of the ELISPOT assay. Each spot is the 'footprint' of a single cell that has secreted IFN-γ in response to the present antigen. Splenocytes were isolated from non-immunised mice (blue), mice immunised with wild-type spores - 168 (red), those immunised with BH108 presenting the ureB antigen (green), and others immunised with BH108 presenting antigen ureB and BHK121 presenting cytokine IL-2 (grey).

The invention is illustrated with the following embodiments which do not restrict the invention in any way. The invention is limited only by the appended claims.

### Example 1:

Preparation of the fusion proteins based on the *H. acinonychis* UreA subunit with the spore coat proteins, and verification of the level of expression and presence of the recombined proteins on the spore surface.

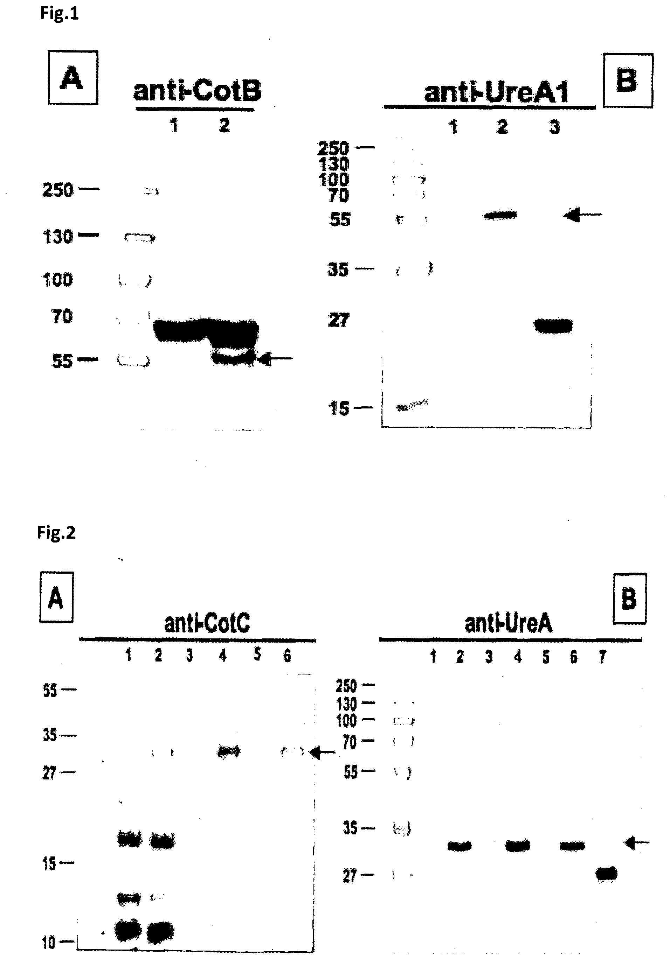

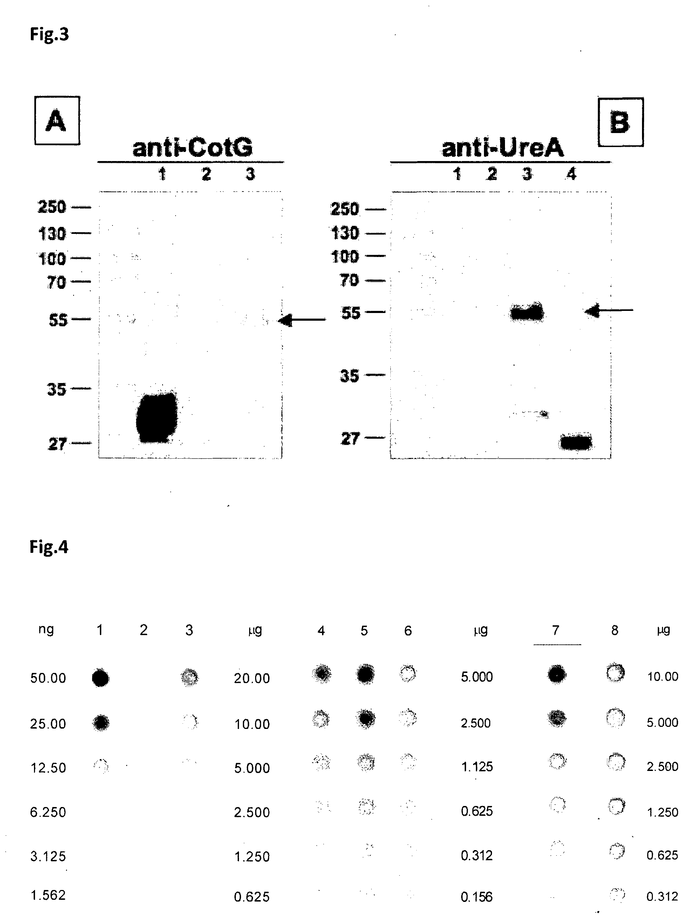

It was established that fusions of proteins CotB, CotC, CotG, CotZ with the *H. acinonychis* UreA subunit and its fragments were effectively presented on the surface of the *B. subtilis* endospores. Moreover, depending on the applied genetic background the number of the recombined proteins isolated from the spore coat was close to or higher than that reported in the literature. The conducted immunofluorescence experiments enabled identification of two types of spores. In one type, the antigen was exposed directly on the surface, whereas in the second type the fusion proteins were inside the coat, hence were not exposed outside the endospores (Fig. 1, Fig.2, Fig.3, Fig.4).

Both types of spores (presenting fragments of the UreA or UreB proteins) were used in the immunological part of the research. The spores where the antigen is not exposed directly on the surface offer more effective antigen protection from degradation on its way along the upper elements of the gastrointestinal tract down to its destination (stomach, intestines), which should consequently enable the attainment of the optimal induction of the immune response.

### Example 2:

### Tests of immunisation of laboratory animals

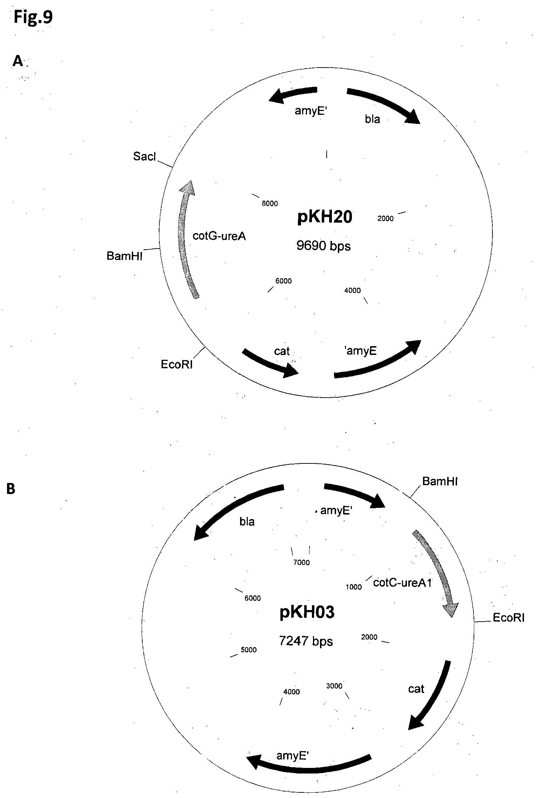

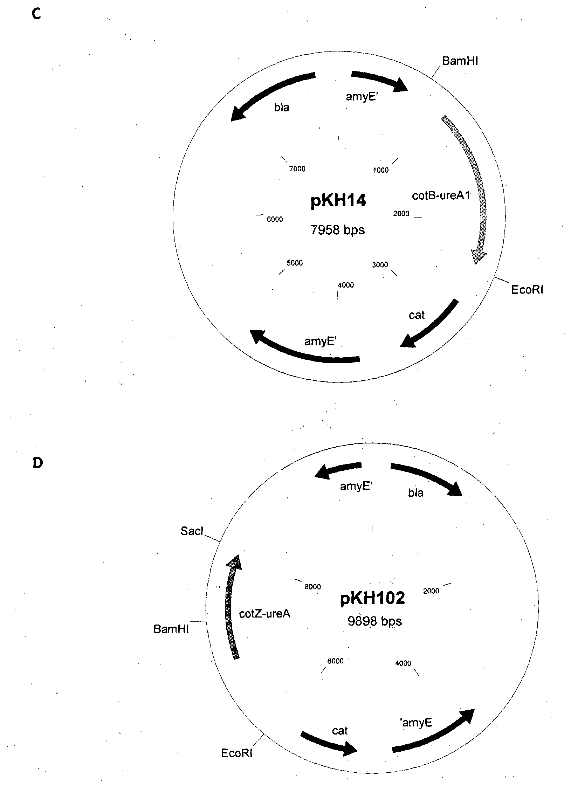

The pDL integration vector was used as the starting point to prepare the fusion protein (CotZ-UreA). The *cotZ* gene from *B. subtilis,* together with the promoter region, was copied under the PCR method using primers: cZ-F 5'- GCT TAG GAT CCA TGA TGA TGT GTA CGA TTG -3' and cY-R 5'- CGT AGC GAA TTC AGT TAT CAC TCT TGT CCT C -3'. Restriction sites (*Bam*HI and *Eco*RI, marked in the primer sequence) were introduced during the PCR reaction facilitating both the process of cloning to the vectors and the formation of the fusion protein. The UreA subunit of the *H. aciononyhis* urease became the second component of the recombined protein. The *ureA* gene was copied under the PCR method using primers: uA-F 5'- GAG GGA TCC ATG AAA CTC ACC CCA AAA G-3' and uA-R 5'- CGC GAG CTC TAG GGC CAT ACA TAG AAA C- 3'. Restriction sites (*Bam*HI and *Sac*I*,* marked red in the primer sequence) were introduced during the PCR reaction facilitating the fusions of the *cotZ* and *ureA* genes in the correct reading frame.
The obtained fusion gene was sequenced so as to verify the attainment of the planned product. The obtained plasmid carrying the fusion gene was called pKH102.
The fusion proteins (CotG-UreA, CotC-UreAl, and CotB-UreAl) were prepared in an analogical manner (Fig.5, Fig.6, Fig.7, Fig.8).
The immediately following step consisted in transformation of the *B. subtilis* cells with the pKH102 plasmid containing the fusion gene. In effect, the recombined gene was incorporated into locus *amyE* of the bacterial chromosome. The obtained bacterial strain was called BKH102. On the other hand, transformation of the *B. subtilis* cells with the pKH20, pKH03, and pKH14 plasmids (Fig.9) containing the recombined genes *cotB-ureA, cotC-ureA1*, and *cotB-ureA1*, enabled obtaining strains BKH20, BKH03, and BKH14. The analysis of the expression level and presence of the recombined proteins on the spore surface using the strains is illustrated in example 1.
In order to reinforce the response attained upon administration of the preparation to laboratory animals, a vector was prepared based on the pDG1662 plasmid, which enabled incorporation of the UreA subunit of the *H. acinonychis* urease into the *B. subtilis* chromosome, in locus *thrC* of the coding gene under the control of a powerful active promoter in the vegetative cells (ribosomal RNA rrnO P2 gene promoter). Administered as vaccine, the spores germinate in the gastrointestinal tract forming vegetative cells, which can express additional quantities of the antigen. The promoter region was copied under the PCR method using primers: rOP2-F 5- GAT GGC T**AA GCT TC**A TGG GTC TCA CCT CCT TGT TC -3' and rOP2-R 5'- GGC GTA GAG **GAA** TTC GAT CTG CAT GAC CAT TAT GAC -3'. Restriction sites (*Hind*III and *Eco*RI, marked in the primer sequence) were introduced during the PCR reaction. Then, the PCR method was employed to copy the *ureA* gene using primers: ruA-F 5- GGC A**TT AAG CT**T AAC TAT GTA G -3' and ruA-R 5'- CTA A**AC ATG T**TC ATT TCT TAC TC -3'. During the PCR reaction restriction sites were introduced (*Hind*III and *Pci*I, marked in the primer sequence) and the control of the ribosomal promoter imposed. The obtained vector was called pKH115.
The subsequent step consisted in transformation of the *B. subtilis* cells strain BKH102 (*amyE::cotZ-ureA*) with plasmid pKH115. In effect, the recombined gene was incorporated into locus *thrC* of the bacterial chromosome. This solution enabled creating a strain that expressed the UreA antigen on the spore surface (fusion protein CotZ-UreA) and in the vegetative cell. The obtained strain was called BKH116 (*amyE::cotZ-ureA*, *thrC::p_{rrnO2}-ureA*)*.* The cultures of the BKH102 and BKH116 strains were analysed for expression of the recombined protein CotZ-UreA and protein UreA using anti-UreA mouse antibodies obtained in our laboratory. The presence of the fusion protein was tested in the protein extracts obtained from the spore coat of the BKH102 strain, and the UreA protein was tested in protein extracts prepared from the vegetative cells of the BKH116 strain. In both cases the western-blotting method was applied, consisting in detection of the specific protein bound to the membrane using enzyme-bound antibodies, where the colour staining reaction enables its location.

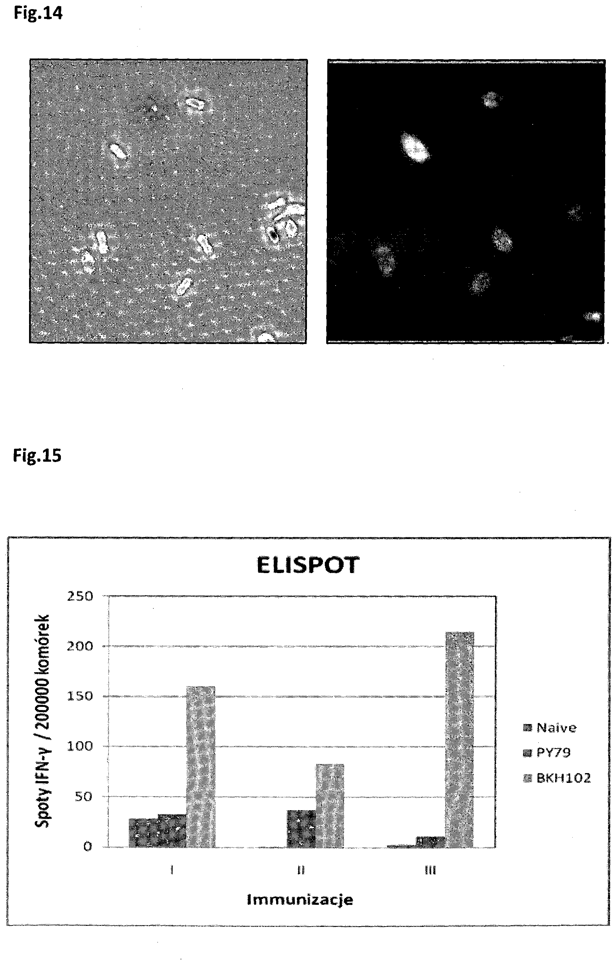

The suitability of the spores carrying the fusion protein in oral immunisation stems from the location of the fusion protein within the spore structures. It is assumed that the fusion proteins present on the spore surface demonstrate a higher potential of stimulating the immune system. The presence of the recombined protein CotZ-UreA on the surface of the spores produced by strain BKH102 was confirmed by the immunoflorescence method (Fig.14, right panel). The visible red fluorescence forming around the spore proves the surface location of the protein in question.

Upon verification of the expression and location of the fusion protein in the spore structures, the procedure turned to purification of the spores in the quantity sufficient to immunise a group of 10 mice. To that end, cultivation to a larger scale was initiated (15 litres of the DSM culture medium), which enabled isolation of an adequate quantity of spores. The spores were purified by rinsing in water and detergents, and then counted in the Thoma chamber. Each batch of the purified spores was tested for the presence of the appropriate fusion proteins on their surface (the data not shown). Spore purification completed the stage of preparing the material used to immunise the laboratory animals (mice) at a later stage. The procedures of immunising the laboratory animals with the created vaccine and assessing the immune response (the degree and type of immune response induction) followed subsequently with assessment of the vaccine effectiveness were conducted on mice of the BALB/c stock (females aged 6 weeks +). The vaccine in nine doses of 1.0 x 10¹⁰ (equivalent to 5mg of spores per dose), containing antigen in the form of *H. acinonychis* urease subunit UreA on the surface, was administered into the stomach with a syringe connected to a polyethylene cannula. The animals were vaccinated on the following days: 0, 3, 5, 14, 16, 18, 30, 32, and 34. On day 56 they were euthanised. The experimental group was made up of 10 animals. The reaction of the immune system was analysed for the humoral and cellular responses. Blood, excreta, and tissue of the animals (the spleen and stomach) served as the material for testing.
The ELISPOT assay (Fig.15) combining the enzyme-linked immunosorbent assay technique with short-term cell cultivation revealed an increased number of cytokines (IFN-y) secreted by splenocytes (immune system cells isolated from the spleen) of the mice immunised with the spores presenting fusion protein CoZ-UreA. The attained result proves activation of the cellular immune response (th1). The fact prognosticates well for the vaccine, since analysis of the information available from the literature indicates that this particular type of the immune response is most effective in elimination of the pathogen.

### Example 3:

Preparation of the fusion proteins based on the *H. acinonychis* UreB subunit with the spore coat proteins, and verification of the level of expression and presence of the recombined proteins on the spore surface.

The pDG364 integration vector was used as the starting point to prepare the fusion protein (CotC-UreB3). The *cotC* gene from *B. subtilis,* together with the promoter region, was copied under the PCR method using primers: cC-F 5'- ACC C**AA GC**T TTG TAG GAT AAA TCG TTT G -3' and cC-R 5'- **GAT ATC** GTA GTG TTT TTT ATG CTT -3'. Restriction sites (*Hind*III and EcoRV, marked in the primer sequence) were introduced during the PCR reaction facilitating both the process of cloning to the vectors and the formation of the fusion protein. The UreB subunit of the *H. aciononyhis* urease became the second component of the recombined protein. A fragment of the *ureB* gene was copied under the PCR method using primers: uB-F 5'- GCT GGT GAT ATC GGC TTT AAA ATC CAC-3' and uB-R 5'- GCA CCT TAC TAG TAA CTT TTG TTG CTT GAG C -3'. Restriction sites (*Eco*RV and *Spe*I, marked red in the primer sequence) were introduced during the PCR reaction facilitating the fusions of the *cotC* and *ureB* genes in the correct reading frame
The obtained fusion gene was sequenced so as to verify the attainment of the planned product. The obtained plasmid carrying the fusion gene was called pKH28 (Fig.16).

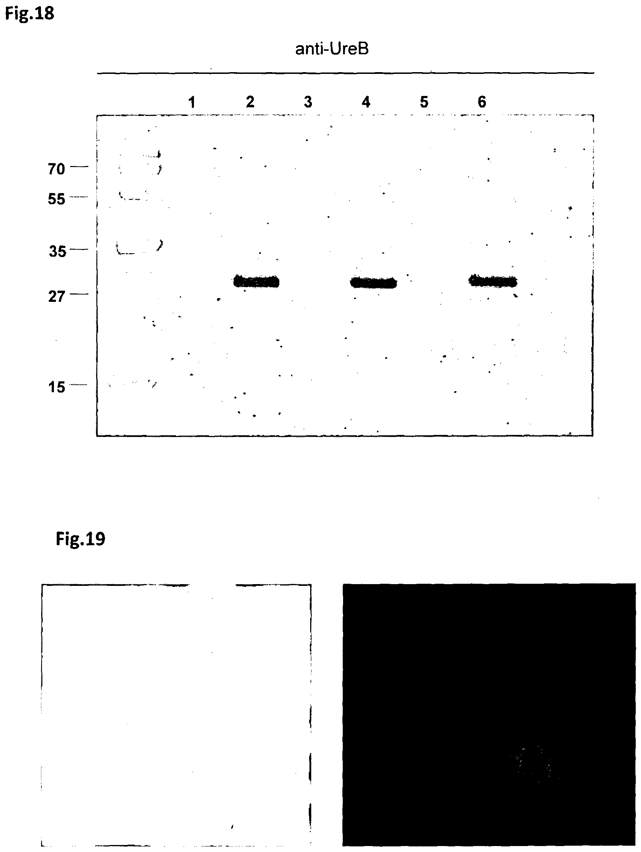

The immediately following step consisted in transformation of the *B. subtilis* cells with the pKH28 plasmid containing the fusion gene. In effect, the recombined gene was incorporated into locus *amyE* of the bacterial chromosome. The obtained bacterial strain was called BKH28.
The culture of the BKH28 strain was analysed for expression of the recombined protein CotC-UreB3 of the mouse anti-UreB antibodies obtained in our laboratory. The presence of the fusion protein was tested in the protein extracts obtained from the spore coat of the BKH28 strain applying the western-blotting method, consisting in detection of the specific protein bound to the membrane using enzyme-bound antibodies, where the colour staining reaction enables its location (Fig.18).

The presence of the recombined protein CotC-UreB3 on the surface of the spores produced by strain BKH28 was confirmed by the immunoflorescence method (Fig.19, right panel).

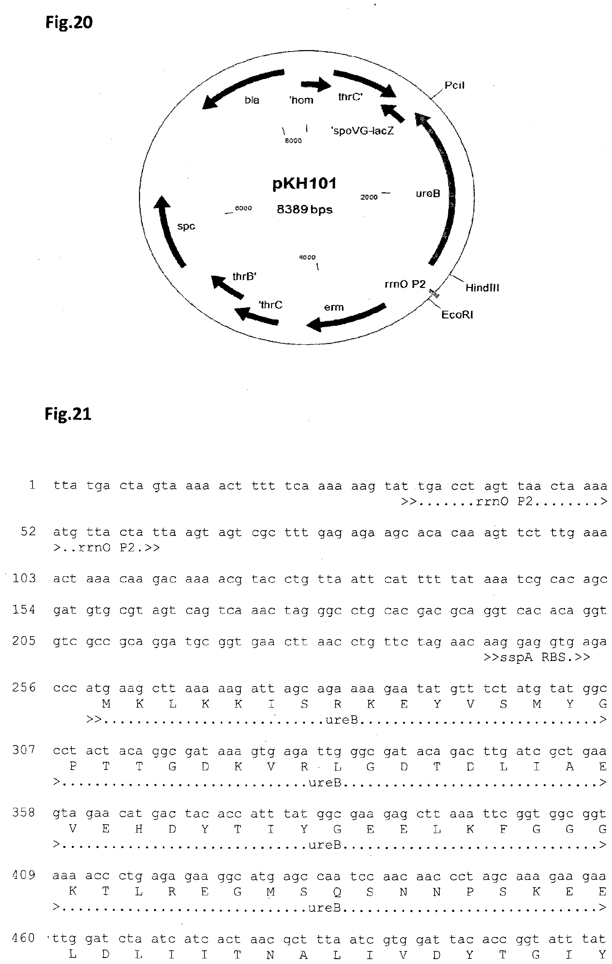

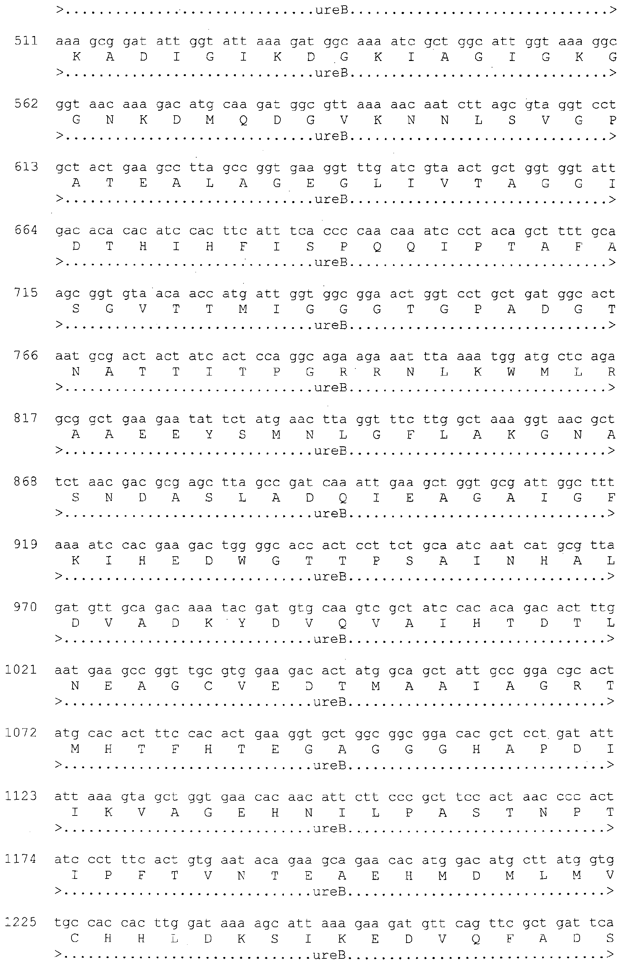

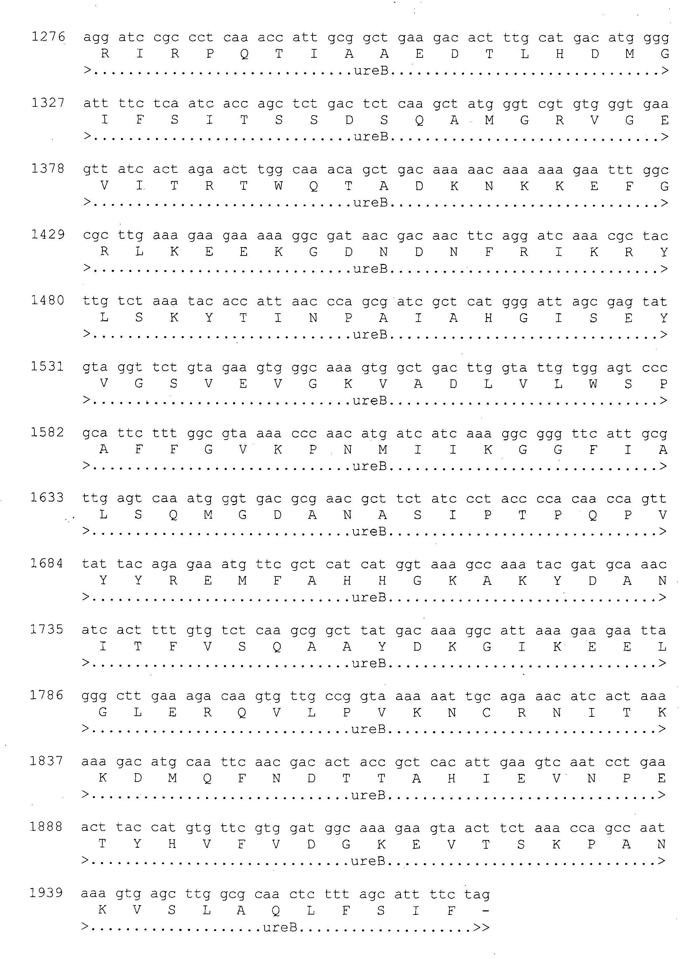

In order to reinforce the response attained upon administration of the spores presenting antigen UreB on the surface to laboratory animals, a vector was prepared based on the pDG1662 plasmid, which enabled incorporation of the UreB subunit of the *H. acinonychis* urease into the *B. subtilis* chromosome, in locus *thrC* of the coding gene under the control of a powerful active promoter in the vegetative cells (ribosomal RNA rrnO P2 gene promoter). Administered as vaccine, the spores germinate in the gastrointestinal tract forming vegetative cells, which can express additional quantities of the antigen. The promoter region was copied under the PCR method using primers: rOP2-F 5- GAT GGC T**AA** GCT TCA TGG GTC TCA CCT CCT TGT TC -3' and rOP2-R 5'- GGC GTA GAG **GAA TTC** GAT CTG CAT GAC CAT TAT GAC -3'. Restriction sites (*Hind*III and *Eco*RI, marked in the primer sequence) were introduced during the PCR reaction. Then, the PCR method was employed to copy the *ureB* gene using primers: uB-F 5'- CCA T**AA GCT** TAA AAA GAT TAG CAG AAA AG -3' and uB-R 5'- CTC C**AC ATG** TAT CCT AGA AAA TGC TAA AG -3'. During the PCR reaction restriction sites were introduced (*Hind*III and *Pci*I, marked red in the primer sequence) and the control of the ribosomal promoter imposed (Fig.21). The obtained vector was called pKH101 (Fig.20).

The subsequent step consisted in transformation of the *B. subtilis* cells strain BKH28 (*amyE::cotC-ureB3*) with plasmid pKH101. In effect, the recombined gene was incorporated into locus *thrC* of the bacterial chromosome. This solution enabled creating a strain that expressed the UreB antigen on the spore surface (fusion protein CotC-UreB3) and in the vegetative cell. The obtained strain was called BKH108 (*amyE::cotC-ureB3*, *thrC::p_{rrnO2}*-*ureB*).

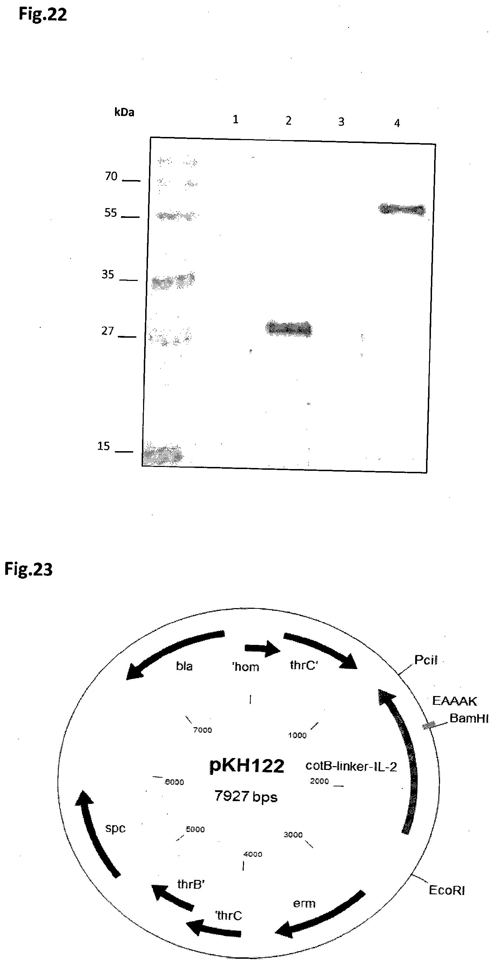

The culture of the BKH108 strain was analysed for expression of the recombined protein CotC-UreB3 and protein UreB using mouse anti-UreB antibodies obtained in our laboratory. The presence of the fusion protein was tested in the protein extracts obtained from the spore coat, and the UreB protein was tested in protein extracts prepared from the vegetative cells. In both cases the western-blotting method was applied, consisting in detection of the specific protein bound to the membrane using enzyme-bound antibodies, where the colour staining reaction enables its location (Fig.22).
The quality of vaccines is frequently attained at the expense of reducing their ability to stimulate specific immune responses. The immunogenicity of the antigen can, however, be enhanced when administered together with adjuvants, which improve the vaccine's effectiveness in many ways. The substances can, e.g. stimulate the cells of the immune system non-specifically and pertain to more efficient absorption and presentation of the antigen. By selecting an appropriate adjuvant one can obtain the immune response of the specific type - Th1 or Th2.
Here, cytokines served as the adjuvant, though instead of stimulating their production with appropriate agents, they were administered directly, together with the antigen. In this way, it was possible to control the antigen processing and APC activation at the level of one and the same cell. In our research we used spores carrying IL-2, in the function of the adjuvant, on their surface in the oral vaccines.

The pDG1663 integration vector was used as the starting point to prepare the fusion protein (CotB-linker-IL-2). The *cotB* gene from *B. subtilis,* together with the promoter region, was copied under the PCR method using primers: cB-F 5'- GC**G GAT CC**G GAT GAT TGA T -3' and cB-R 5'- GAT **GAA TTC** ACG GAT TAG -3'. Restriction sites (*Bam*HI and *Eco*RI, marked red in the primer sequence) were introduced during the PCR reaction facilitating both the process of cloning to the vectors and the formation of the fusion protein. The other component of the recombined protein, cytokine IL-2, was synthesised (EUROGENTEC) together with the sequences coding the peptide linkers sequenced GGGEAAAKGGG at the beginning and end of the formed protein. Then, gene IL-2 (containing the linker coding sequence 5' at the end) was copied under the PCR method using primers: IL2link-F 5'- GCT TCA CAT GTT TAC GTC AGT GTA GAG ATG ATA GAT TGG C -3' and IL2link-R 5'- CAT AT**G GAT** CCG GTG GAG GAG AAG CAG CAG CG -3'. Restriction sites (Pcil BamHI, marked red in the primer sequence) were introduced during the PCR reaction facilitating the fusions of the *cotB-linker-IL2* genes in the correct reading frame (Fig.24).

The obtained fusion gene was sequenced so as to verify the attainment of the planned product. The obtained plasmid carrying the fusion gene was called pKH122 (Fig.23).

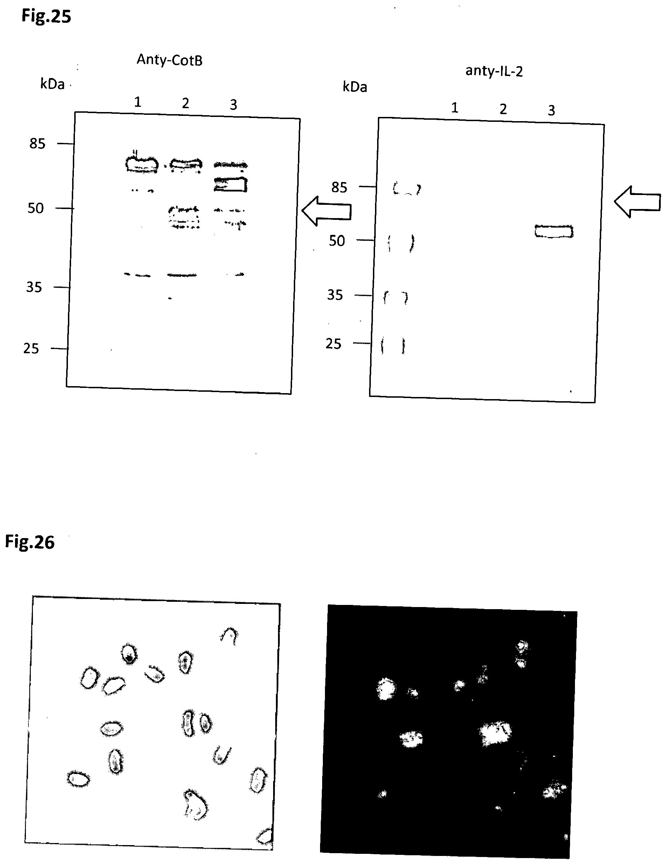

The subsequent step consisted in transformation of the *B. subtilis* cells with the pKH122 plasmid. In effect, the recombined gene *cotB-linker-IL-2* was incorporated into locus *thrC* of the bacterial chromosome. The obtained bacterial strain was called BKH121.
The purified spores produced by the above strain were tested for the presence of recombined coat proteins using mouse anti-IL-2 antibodies under the western-blotting method (Fig. 25).

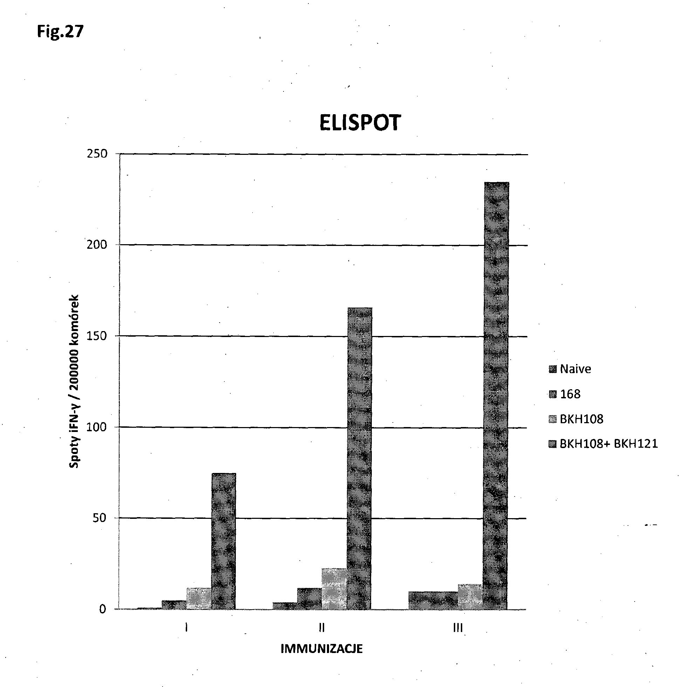

The quantity of the recombined proteins located in the outer spore coat was assessed by the dot-blotting technique where proteins are applied directly on the membrane and then detected by antibodies. The assays revealed that the quantity of the recombined proteins CotB-linker-IL-2 isolated from the spore coat stood at 1.1 x 10⁴ molecules/spore.
The usefulness of the spores carrying fusion proteins in oral immunisation is due to the location of the fusion protein in the spore structures. It is assumed that the fusion proteins present on the spore surface demonstrate a higher potential of stimulating the immune system. The presence of the recombined proteins on the surface of the spores was confirmed by the immunoflorescence method. The fusion of CotB-linker-IL-2 is deposited on the surface of the recombined spores (Fig.26).
Upon verification of the expression and location of the fusion proteins in the spore structures, the procedure turned to purification of the spores in the quantity sufficient to immunise a group of 10 mice. To that end, cultivation to a larger scale was initiated (15 litres of the DSM culture medium per strain), which enabled isolation of an adequate quantity of spores. The spores were purified by rinsing in water and detergents, and then counted in the Thoma chamber. Each batch of the purified spores was tested for the presence of the appropriate fusion proteins on their surface (the data not shown). Spore purification completed the stage of preparing the material used to immunise the laboratory animals (mice) at a later stage. The procedures of immunising the laboratory animals with the created vaccine and assessing the immune response (the degree and type of immune response induction) followed subsequently with assessment of the vaccine effectiveness were conducted on mice of the BALB/c stock (females aged 6 weeks +). The vaccine of recombined spores of the BKH108 (*amyE::cotC-ureB3 thrC::rnnOP2-UreB*) and BKH121 (thrC::cotB-linker-IL-2) strains, at 1.0 x 10¹⁰, i.e. 2.0 x 10¹⁰ (equivalent to 10mg of spores per dose) was administered into the stomach with a syringe connected to a polyethylene cannula. The animals were vaccinated on the following days: 0, 3, 5, 14, 16, 18, 30, 32, and 34. On day 56 they were euthanised. The experimental group was made up of 10 animals. The reaction of the immune system was analysed for the humoral and cellular responses. Blood, excreta, and tissue of the animals (the spleen and stomach) served as the material for testing.
The ELISPOT assay combining the enzyme-linked immunosorbent assay technique with short-term cell cultivation revealed an increased number of cytokines (IFN-y) secreted by splenocytes (immune system cells isolated from the spleen) of the mice immunised with the spores presenting fusion protein *cotC-ureB3* in the presence of spores presenting cytokine IL-2 on the surface (Fig. 27). The attained result proves activation of the cellular immune response (Th1). The fact prognosticates well for further work on the vaccine development, since analysis of the information available from the literature indicates that this particular type of the immune response is most effective in elimination of the pathogen.

## Claims

1. An oral vaccine comprising:
(a) spores of *Bacillus subtilis* expressing on their surface the CotC protein fused to the antigen UreB3 of *Helicobacter pylori* or *Helicobacter acinonychis,* and
(b) spores of *Bacillus subtilis* expressing on their surface the CotB protein fused to the cytokine IL2.

2. The vaccine of the preceding claim, wherein the said CotC-UreB3 fusion protein is **characterized by** the sequence depicted in Fig.17, namely:

MGYYKKYKEEYYTVKKTYYKKYYEYDKKDYDCDYDKKYDDYDKKYYDHDKKDYDYVVEYKKHK
KHYDIKYTINPAIAHGISEYVGSVEVGKVADLVLWSPAFFGVKPNMIIKGGFIALSQMGDANA
SIPTPQPVYYREMFAHHGKAKYDANITFVSQAAYDKGIKEELGLERQVLPVKNCRNITKKDMQ
FNDTTAHIEVNPETYHVFVDGKEVTSKPANKVSLAQLFSIF-

3. The vaccine of any one of the preceding claims, **characterized in that** is produced in powder or suspension form.

4. The vaccine of any one of the preceding claims, for use in vaccination against *Helicobacter pylori.*

## Patentansprüche

1. Schluckimpfstoff, bestehend aus:
(a) Sporen von *Bacillus subtilis,* die auf ihrer Oberfläche das mit dem Antigen UreB3 von *Helicobacter pylori* oder *Helicobacter acinonychis* fusionierte CotC-Protein exprimieren,
und
(b) Sporen von *Bacillus subtilis,* die auf ihrer Oberfläche das mit dem Zytokin IL2 fusionierte CotB-Protein exprimieren.

2. Impfstoff nach dem vorhergehenden Anspruch, worin das besagte CotC-UreB3-Fusionsprotein durch die in Abb. 17 dargestellte Sequenz gekennzeichnet ist, nämlich:

MGYYKKYKEEYYTVKKTYYKKYYEYDKKDYDCDYDKKYDDYDKKYYDHDKKDYDYVVEYKKHK
KHYDIKYTINPAIAHGISEYVGSVEVGKVADLVLWSPAFFGVKPNMIIKGGFIALSQMGDANA
SIPTPQPVYYREMFAHHGKAKYDANITFVSQAAYDKGIKEELGLERQVLPVKNCRNITKKDMQ
FNDTTAHIEVNPETYHVFVDGKEVTSKPANKVSLAQLFSIF-

3. Impfstoff nach einem der vorstehenden Ansprüche, **dadurch gekennzeichnet, dass** er in Form eines Pulvers oder einer Suspension hergestellt wird.

4. Impfstoff nach einem der vorhergehenden Ansprüche zur Verwendung bei der Impfung gegen *Helicobacter pylori.*

## Revendications

1. Un vaccin oral comprenant :
(a) des spores de *Bacillus subtilis* exprimant à leur surface la protéine CotC fusionnée à l'antigène UreB3 de *Helicobacter pylori* ou de *Helicobacter acinonychis,*
et
(b) des spores de *Bacillus subtilis* exprimant à leur surface la protéine CotB fusionnée à la cytokine IL2.

2. Vaccin selon la revendication précédente dans lequel ladite protéine de fusion CotC-UreB3 est **caractérisée par** la séquence présentée sur la figure 17, à savoir:

MGYYKKYKEEYYTVKKTYYKKYYEYDKKDYDCDYDKKYDDYDKKYYDHDKKDYDYVVEYKKHK
KHYDIKYTINPAIAHGISEYVGSVEVGKVADLVLWSPAFFGVKPNMIIKGGFIALSQMGDANA
SIPTPQPVYYREMFAHHGKAKYDANITFVSQAAYDKGIKEELGLERQVLPVKNCRNITKKDMQ
FNDTTAHIEVNPETYHVFVDGKEVTSKPANKVSLAQLFSIF-

3. Vaccin selon l'une quelconque des revendications précédentes **caractérisé en ce qu'**il est produit sous forme de poudre ou de suspension.

4. Vaccin selon l'une quelconque des revendications précédentes, destiné à être utilisé dans la vaccination contre *Helicobacter pylori.*
